# EUROPEAN PATENT APPLICATION

(11) **EP 1 801 115 A1**
(43) Date of publication of application: **27.06.2007**
(21) Application number: 05028301.9
(22) Date of filing: 23.12.2005
(51) Int. Cl.: C07K 5/12, A61K 31/00, A61P 35/00

(54) **Cyclic peptides and their use in treating advanced stage neuroblastoma**

(71) Applicant: Deutsches Krebsforschungszentrum Stiftung des öffentlichen Rechts, 69120 Heidelberg (DE)
(72) Inventor: Witt, Olaf, 69198 Schriesheim (DE); Deubzer, Hedwig Elisabeth, 69121 Heidelberg (DE); Westermann, Frank, 69221 Dossenheim (DE); Roenndahl, Gabi, 32427 Minden (DE); Heinrich, Ralf, 37077 Göttingen (DE); Ehemann, Volker, 69190 Walldorf (DE)
(74) Representative: Huhn, Michael

(57) **Abstract**

The present invention relates to uses of compounds having a structure as shown by formula (I) for the manufacture of a pharmaceutical composition for the treatment of neuroblastoma. Moreover, the present invention encompasses methods of treatment for said diseases.

## Description

The present invention relates to uses of compounds having a structure as shown by formula (I) for the manufacture of a pharmaceutical composition for the treatment of neuroblastomas. Moreover, the present invention encompasses methods of treatment for such diseases.

The pediatric neuroblastoma in its advanced stage (stage IV) is usually lethal. Although chemotherapy, surgery and radiation are available, statistically, some 70 % of the affected children die. The therapies available so far often are accompanied with severe side effects resulting in lethality. The current therapy of neuroblastoma pivotally comprises chemotherapy, radiation therapy, surgery, radionuclear therapeutic approaches as well as antibody or retinoic acid based therapies (F. Berthold (head of studies), NB2004 Trial Protocol for Risk Adapted Treatment of Children with Neuroblastoma, Children's University Hospital Cologne, Dept. Pediatric Oncology, Cologne, Germany). In 70% to 90% of the cases, using a risk adopted therapeutic approach, localized stages of the diseases may be either treated by surgery alone or by a combination of surgery and radiation (F. Berthold et al., 2003, Cancer Lett 197: 11-17) (Comment: Detailed references are missing in this document.). Half of the children suffering from neuroblastoma, however, show upon diagnosis metastasis or a genetic amplification of the *MYCN* oncogene. This group of patients has a poor prognosis and a (5 year period) survival rate of merely 33 % even though treated by multiple approaches, such as surgery, chemotherapy, high dosage chemotherapy including stem cell transplantation, radiation, iodine-131 metaiodobenzylguanidine (¹³¹I-MIBG) therapy and anti-GD2 antibody therapy (Berthold et al., 2003, Cancer Lett 197: 11-17). However, administration of the differentiation inducing agent retinoic acid could somewhat improve the prognosis for the patients of the risk group mentioned above (Matthay et al., 1999, N Engl J Med 341: 1165-1173).

A drawback of the current standard therapy for high risk neuroblastoma using surgery, chemotherapy, high dosage chemotherapy including stem cell transplantation, radiation and ¹³¹I-MIBG therapy is the poor efficacy accompanied by the high lethality rate of approx. 70 %. Moreover, serious side effects have been regularly encountered including toxicity for the mucous membranes, damages of kidney, inner ear and heart as well as, most importantly, severe bone marrow depression resulting in the need of frequent transfusions and in fulminant infections leading to lethal sepsis.

It is, therefore, an object of the present invention to provide means and methods for treating neuroblastoma. Preferably, the following requirements shall be complied with:
- The anti-tumoral mode of action shall not be based on the cytotoxic principles of traditional chemotherapy because this will not improve the results of the therapy.
- Only neuroblastoma cells shall be affected but not "normal" non-transformed cells of the body.
- *MYCN* oncogene amplified neuroblastoma shall be treatable. Specifically, *MYCN-*amplified neuroblastoma cells are particularly aggressive and patients suffering from *MYCN* amplified tumours usually have a poor prognosis with a survival rate of merely 20 % to 30 %.
- A pharmacologically suitable dosage regimen *in vivo* should be achieved.

This object is attained by a compound according to the general formula (I)

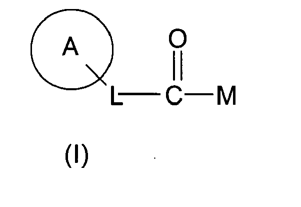

In the most general meaning classified below under A, the symbols have the following meanings:
**A:**
   A is a 9- to 15-membered heterocycle optionally carrying one or more double bonds, and comprising 3 to 5 units of the type -D-E-G- wherein
   D is selected from -N(R¹)-, -O-, and -S(O)(R²R³)ₘ-; and/or
   E is selected from methylene -CH₂-, ethylene -CH₂CH₂- and propylene -CH₂-CH₂-CH₂-, wherein in the methylene, ethylene and propylene groups one or more H atoms can be replaced by: linear and branched C₁- to C₁₀ alkyl groups, which are optionally substituted by one or more substituents from the group -OH, F, -SH, -SCH₃, -NH₂, -NHC(NH)NH₂, - C(O)OH, -C(O)NH₂, C₆H₅, C₆H₄OH, indolyl, imidazolyl or a salt thereof; and linear and branched C₂- to C₁₀ alkylene groups, which are optionally substituted by one or more OH and/or one or more F; or, in case 2 or more even-numbered substituents are present, the named alkyl or alkylene group forms together with a further alkyl or alkylene group on the same or a different carbon atom a 5- to 7-membered cycle; or the named alkyl and alkylene groups form together with the group R¹ a 5- to 7-membered cycle which includes the C and the N atom to which the named groups are attached; and/or
   G is a keto group -C(O)- or a thioketo group -C(S)-; and/or
      wherein the units -D-E-G- not attached to L can be fully or partially identical with or entirely different from each other; and/or
      the cycle A can comprise one or more double bonds; and/or
   L is attached to D or E in one unit -D-E-G- and is a linear C₃- to C₅-alkyl group in which one or more carbon atoms may be replaced by non adjacent O atoms, and wherein one or more H atom in the alkyl chain may be substituted by one or more alkyl groups; and/or
   M is selected from epoxy groups 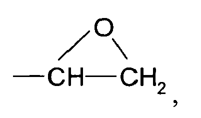 hydroxyamino groups -N(OH)H, hydroxymethyl groups -CH₂OH, 1-fluoroethyl groups - CHFCH₃ and 1-chloroethyl groups -CHClCH₃; and/or
   R¹ is selected from: H; linear and branched C₁- to C₁₀ alkyl groups, which are optionally substituted by one or more OH and/or one or more F; and linear or branched C₂- to C₁₀ alkylene groups which are optionally substituted by one or more OH and/or one or more F; or R¹ forms together with one alkyl or alkylene group in E a 5- to 7-membered cycle which includes the C and the N atom to which the named groups are attached; and/or
   R² and R³ are independently of each other selected from: H; linear and branched C₁- to C₁₀ alkyl groups which are optionally substituted by one or more OH and/or one or more F; and linear and branched C₂- to C₁₀ alkylene groups, which are optionally substituted by one or more OH and/or one or more F; or R² and R³, together with the sulfur atom to which they are attached, form a 5- to 7-membered cycle; or one of R², R³ forms together with one alkyl or alkylene group in E a 5- to 7-membered cycle, which includes the C and the N atom to which the named groups are attached; and/or
   m is 0 or 1,
      and/ or a pharmaceutically acceptable salt thereof.
   L is always attached to E in one of the units -D-E-G-, which unit has to be different from the other units -D-E-G-.
   The cycle A comprises 3, 4 or 5 amino acids. Suitable amino acids are known to the person skilled in the art and comprise natural amino acids and synthetic amino acids. Natural amino acids are those occurring in nature and comprise: glycine, alanine, serine, cysteine, phenylalanine, tyrosine, tryptophane, threonine, methionine, valine, proline, leucine, isoleucine, lysine, arginine, histidine, aspartic acid, asparagine, glutamic acid, glutamine. The named amino acids are found in the L form in nature. In the context of the present invention, the above referenced natural amino acids in the L-form and in the D-form are preferred.
   The double bonds in the cycle A may be a result of enol forms resulting from tautomerism. In case enol forms result from tautomerism between the keto group and a neighbouring -NH or a -CH function, the enol forms may be stabilised or fixed by alkylation. Suitable alkylation methods are known to the person skilled in the art.
   The present invention also includes prodrugs of the compounds of formula (I). Examples for suitable prodrugs include molecules according to formula (I) as defined beforehand, and wherein the epoxy function is replaced by an appropriate precursor function therof, e.g. a vicinal diol function, a chlorohydrine function -CH(OH)(Cl)CH- or an olefinic double bond.
   Preferred embodiments are classified below under B:
**B:**
   A is a 11- to 13-membered cycle; comprises 4 units of the type -D-E-G- , wherein
   D is NR¹; and/or
   E is -CH₂-, wherein one or both H atoms can be replaced by: a linear or branched C₁- to C₄ alkyl group, which are optionally substituted by one or more substituents from the group - OH, F, -SH, -SCH₃, -NH₂, -NHC(NH)NH₂, -C(O)OH, -C(O)NH₂, C₆H₅, C₆H₄OH, indolyl, imidazolyl or a salt thereof; and/or a linear or branched C₂- to C₄ alkylene group, which is optionally substituted by one or more OH and/or one or more F; or the named alkyl and alkylene groups form together with the group R¹ a 5- to 7-membered cycle which includes the C and the N atom to which the named groups are attached; and/or
   G is a keto group -C(O)-; and/or
      wherein the units -D-E-G- not attached to L can be fully or partially identical with or entirely different from each other; and/or
      the cycle A can comprise one or more double bonds; and/or
   R¹ is selected from: H; linear and branched C₁- to C₄ alkyl groups, which are optionally substituted by one or more OH and/or one or more F; and linear and branched C₂- to C₄ alkylene groups which are optionally substituted by one or more OH and/or one or more F; or R¹ forms together with one alkyl or alkylene group in E a 5- to 7-membered cycle, which includes the C and the N atom to which the named groups are attached, and/or
   L is attached to E in one unit -D-E-G- and is a linear C₃- to C₅-alkyl group in which one or more carbon atoms may be replaced by non adjacent O atoms, and wherein one or more H atom in the alkyl chain may be substituted by one or more alkyl groups; and/or
   M is selected from epoxy groups 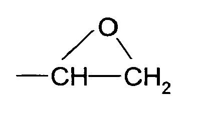 and hydroxyamino groups -N(OH)H.
   The cycle A comprises 4 natural amino acids in the L- or D-form, selected from those cited beforehand.
   Even more preferred embodiments are classified below under C:
**C**:
   A is a 12-membered cycle and comprises 4 units of the type -D-E-G- , wherein
   D is NR¹; and/or
   E is -CH₂-, wherein in one or two units -D-E-G-, one or both H atoms can be replaced by: a linear or branched C₁- to C₄ alkyl group and/or a linear or branched C₂- to C₄ alkylene group; and wherein in only one unit -D-E-G-, one H atom in -CH₂- is replaced by a linear or branched C₁- to C₄ alkyl group or a linear or branched C₂- to C₄ alkylene group, with the said alkyl or alkylene group forming together with R¹ in the neighbouring NR¹ group a 5-to 7-membered cycle, which includes the C and the N atom to which the named groups are attached; and/or
   G is a keto group -C(O)-; and/or
      at least 2 units -D-E-G- are different from each other; and/or
      the cycle A can comprise one or more double bonds; and/or
   R¹ is selected from: H; linear and branched C₁- to C₄ alkyl groups, which are optionally substituted by one or more OH and/or one or more F; and linear and branched C₂- to C₄ alkylene groups, which are optionally substituted by one or more OH and/or one or more F; and wherein in exactly one unit -D-E-G-, R¹ forms together with the said alkyl or alkylene in the neighbouring -CH₂- group a 5- to 7-membered cycle, which includes the C and the N atom to which the named groups are attached; and/or
   L is attached to E in one unit -D-E-G- and is a linear C₃- to C₅-alkyl group, and wherein one or more H atom in the alkyl chain may be substituted by one or more alkyl groups; and/or
   **M** is selected from epoxy groups 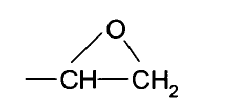 and hydroxyamino groups -N(OH)H; and/or
      the cycle A comprises 4 natural amino acids in the L- or D-form.
   Embodiments, which are still preferred over those under C, are classified below under D:
**D:**
   A is a 12-membered cycle and comprises 4 units of the type -D-E-G- , wherein
   D is NR¹; and/or
   E is -CH₂-, wherein in one or two units -D-E-G-, one or both H atoms can be replaced by: a methyl or ethyl group; and wherein in only one unit -D-E-G-, one H atom in -CH₂- is replaced by a linear or branched C₁- to C₄ alkyl group or a linear or branched C₂- to C₄ alkylene group, and wherein the said alkyl or alkylene group forms together with R¹ in the neighbouring NR¹ group a 5- to 7-membered cycle which includes the C and the N atom to which the named groups are attached;
   G is a keto group -C(O)-; and/or
      wherein at least 2 units -D-E-G- are different from each other; and/or
      the cycle A can comprise one or more double bonds; and/or
   R¹ is H or a linear or branched C₁- to C₄-alkyl group in three of the units -D-E-G-; and wherein in the other unit -D-E-G, R¹ forms together with the said alkyl or alkylene in the neighbouring -CH₂- group a 5- to 7-membered cycle, which includes the C and the N atom to which the named groups are attached; and/or
   L is attached to E in one unit -D-E-G- and is a C₃- to C₅-alkyl group, and wherein one or more H atom in the alkyl chain may be substituted by one or more alkyl groups; and/or
   M is selected from epoxy groups 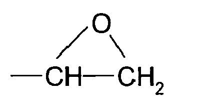 and hydroxyamino groups -N(OH)H; and/or
      the cycle A comprises 4 natural amino acids in the L- or D-form.
   Within the definitions made beforehand for embodiments A, B, C and D, the individual meanings for each substituent or each group are not construed to be restricted to the embodiment under which it is mentioned, i.e. a definition cited under C my be combined with the definitions made under e.g. A, B, or D. The entire combination of all the definitions for each substituent made under each of A, B, C and D is however preferred.
   As an example, for the compounds according to the general formula (I), reference is made to formula (II), i.e. *Helminthosporium carbonum* toxin (HC-Toxin). 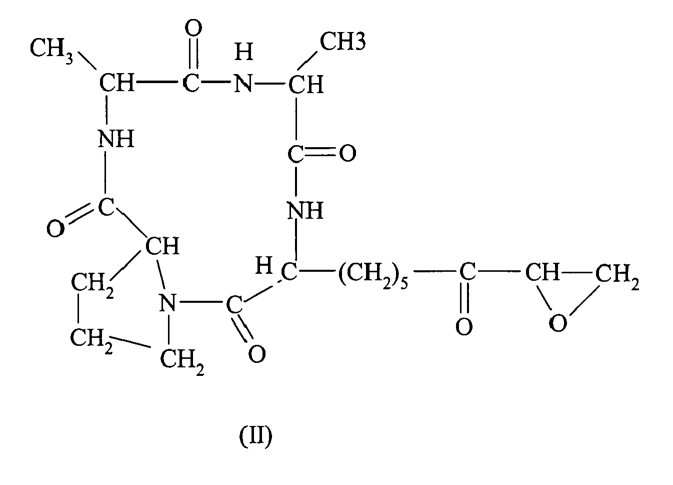 The compound of the general formula (II) is known as such and does as a compound not belong to the present invention, only with respect to its particular pharmacological and biological properties, as laid out hereinafter.
   In a further embodiment, the present invention relates to a compound according to formula (I) as defined above in general form or in preferred embodiments, or a pharmaceutically acceptable salt thereof, for use as medicament. In still a further embodiment, the present invention relates to a compound according to formula (I) as defined above in general form or in preferred embodiments, or a pharmaceutically acceptable salt thereof, for the manufacture of a pharmaceutical composition for the treatment of neuroblastoma.

The term "pharmaceutical composition" is sometimes referred to as "pharmaceutical" or "medicament" hereinafter or in the prior art. Said terms shall have the same meaning and may be used interchangeably.

The term "neuroblastomas" or "neuroblastoma" encompasses all kinds of neuroblastomas. Specifically, all stages of a neuroblastoma are comprised by the term. Staging is carried out, preferably, according to the International Neuroblastoma Staging System (INSS) (Brodeur et al., 1993, J Clin Oncol 11: 1466-1477). In principle, this surgical based staging distinguishes 5 basic stages of neuroblastoma: Stage I: Localized tumour confined to the area of origin. Complete gross resection with or without microscopic residual disease; identifiable ipsilateral and contralateral lymph node negative for tumour.. Stage II: Unilateral tumour with incomplete gross resection; identifiable ipsilateral and contralateral lymph node negative for tumour (stage II a), with ipsilateral lymph node positive for tumour, identifiable contralateral lymph node negative for tumour (stage II b). Stage III: Tumour infiltrating across the midline with or without regional lymph node involvement; or unilateral tumour with contralateral lymph node involvement or midline tumour with bilateral lymph node involvement. Stage IV: Dissemination of tumour to distant lymph nodes, bone marrow, liver, or other organs except as defined in stage IVS. Stage IVS: Localized primary tumour as defined for stage 1 or 2 with dissemination limited to liver, skin, and bone marrow (referred from: Neuroblastoma by Cheung and Cohn, Springer 2005, table 7.5.). Preferably, neuroblastomas as referred to herein are pediatric or childhood neuroblastomas which are in an advanced stage, i.e. stage III, IV or IVS. More preferably, the neuroblastomas to be treated in accordance with the use of the present invention are stage IV neuroblastomas or neuroblastomas having the *MYCN* oncogene amplified, most preferably stage IV neuroblastomas having the *MYCN* oncogene amplified.

Whether a subject suffers from any one of the aforementioned neuroblastomas can be diagnosed by techniques well known in the art. For example, the neuroblastomas may be diagnosed based on accompanying symptoms which are described above. The following tests and procedures may be used to determine the stage: Bone marrow aspiration and biopsy: The removal of a small piece of bone and bone marrow by inserting a needle into the hipbone or breastbone. A pathologist views both the bone and the bone marrow samples under a microscope to look for signs of cancer. Tumour and lymph node biopsy: The removal of all or part of a tumour or lymph node. A pathologist views the tissue under a microscope to look for cancer cells. One of the following types of biopsies may be done: Excisional biopsy, i.e. removal of an entire tumour or lymph node; Incisional biopsy or core biopsy, i.e. removal of part of a tumour or lymph node using a wide needle; Needle biopsy or fine-needle aspiration, i.e. removal of a sample of tissue or fluid from a tumour or lymph node using a thin needle; CT scan (CAT scan); MRI (magnetic resonance imaging) sometimes also called nuclear magnetic resonance imaging (NMRI); X-rays of the chest, bones, and abdomen; Ultrasound (sonogram); or Radionuclide scan: A procedure to find areas in the body where cells, such as cancer cells, are dividing rapidly. A very small amount of radioactive material is swallowed or injected into a vein and travels through the bloodstream. The radioactive material collects in the bones or other tissues and is detected by a radiation-measuring device.

Given the strong association between MYCN oncogene amplification and poor clinical outcome, determination of MYCN oncogene status in neuroblastoma tumours prior to starting therapy has become an international standard and can be carried out as described in (Schwab et al., 2003, Lancet Oncol, 4: 472-480. The MYCN amplification can be determined by standard molecular biology techniques such as PCR-based assays.

The compounds of the general formula (I) and, preferably, *Helminthosporium carbonum* toxin (HC-Toxin), elicit antitumoral effects in nanomolar concentrations via their capability of inducing differentiation, growth arrest, and apoptosis. "Normal" non-transformed body cells, such as primary skin fibroblasts, however, are not affected by the said compounds. Thus, the compounds having a structure as shown in formula (I) improve the success rate of the therapy while reducing the side effects accompanying the prior art therapeutic approaches.

The compounds having a structure as shown in formula (I) induce differentiation, growth arrest, and apoptosis of neuroblastomas cells in nanomolar concentrations. The antiproliferative effects are particularly strong upon *MYCN* amplified advanced stage neuroblastoma, which are known to harbour particularly aggressive tumour behaviour. However, the compounds do, advantageously, not elicit any effect on "normal" non-transformed body cells, such as primary skin fibroblasts.

The compounds of the general formula (I) differ with respect to their mode of action from other chemotherapeutic agents. Specifically, they induce differentiation in neuroblastoma cells. This is particularly advantageous over the use of other chemotherapeutic agents because said agents do not increase the survival rates of patients suffering from neuroblastoma even if administered in high dosages.

Moreover, the compounds are specific for the tumours because they elicit no effects on healthy body cells, e.g., "normal" non-transformed skin fibroblast. Accordingly, there are no or only minor side effects to be expected.

Amplification of the *MYCN* oncogene results in high proliferation rates and aggressive tumour behaviour. Such neuroblastomas have a poor prognosis as discussed elsewhere in the specification. It is known that the chemotherapeutic agents available so far treat those tumours with a significantly lower efficacy than MYCN-single copy ("non-amplified") tumours. The compounds of the present invention however allow to treat in particular those tumours efficiently due to their strong antiproliferative, apoptosis- and differentiation-inducing effects upon *MYCN* amplified neuroblastoma cells.

The compounds having a structure as shown in the general formula (I) are more potent than other known histone deacetylase inhibitors. For example, the histone deacetylase inhibitor valproic acid is only efficient in millimolar concentrations, which are not achievable *in vivo.* Other histone deacetylase inhibitors such as trichostatin A are known to exhibit strong cytotoxic effects. The differentiation inducing agent *all-trans* retinoic acid has to be administered in a 1000-fold higher concentration compared to HC-toxin, acts differently as compared with compounds of the general formula (I) and causes side effects known as the retinoic acid syndrome.

As a result of the differentiation induced by the compounds of the present invention, the tumour cells develop a neuronal phenotype. Neuronal differentiation markers such as neurofilament 3 (150 kDa medium) *(NEF3),* microtubulin associated protein 2 (*MAP2*), tyrosine hydroxylase (*TH*), dopamine, synaptophysin (*SYP*) and synapsin 1 (*SYN1*) are expressed. Simultaneously, growth arrest is induced by blocking the cell cycle at the G0/1 and G2/M transition. Moreover, apoptosis is induced in the said cells.

As discussed above, the compounds of the present invention have no effect upon "normal" non-transformed skin fibroblasts in concentrations, which elicit anti-tumoral effects upon neuroblastoma cells, such as the aforementioned induction of differentiation, growth arrest and apoptosis. Specifically, growth, cell cycle and viability of "normal" non-transformed body cells such as primary skin fibroblasts are not affected by the compounds.

The compounds having a structure as shown in the general formula (I) were found to inhibit proliferation, induce apoptosis and differentiation of *MYCN* non-amplified and physiologically *MYCN* amplified neuroblastoma cells (Be(2)-C) with high efficacy. Moreover, the same strong effects were observed for stably transfected WAC2 cells expressing the *MYCN* oncogene and for neuroblastoma cells, which express said oncogene under the control of a tetracycline inducible system (Tet21N).

The compounds having a structure as shown in the general formula (I) elicit the aforementioned antitumoral effects in an extremely low dosage already, i.e. at nanomolar concentrations (5-20 nM), in various tumour cells. The mode of action of the compounds apparently encompasses inhibition of histone deacetylases. This finding is supported by the observation that treatment of *MYCN* single copy and amplified neuroblastoma cells with a compound of the present invention in nanomolar concentration (e.g. 15 nM) leads to the accumulation of acetylated H4-histones within one to six hours being an indicator for a histone deacetylase inhibitor. Moreover, in comparison to other known histone deacetylase inhibitors, the compounds having a structure as shown in the general formula (I) may be more potent than the said other inhibitors, in the case of suberoylanilide hydroxamic acid (SAHA) up to 60-fold and in the case of valproic acid up to 100000-fold.

The present invention includes all stereoisomeric forms of the compounds of the formula (I). Centers of asymmetry that are present in the compounds of formula (I) all independently of one another have S configuration or R configuration. The invention includes all possible enantiomers and diastereomers and mixtures of two or more stereoisomers, for example mixtures of enantiomers and/or diastereomers, in all ratios. Thus, compounds according to the present invention which can exist as enantiomers can be present in enantiomerically pure form, both as levorotatory and as dextrorotatory antipodes, in the form of racemates and in the form of mixtures of the two enantiomers in all ratios. In the case of a cis/trans isomerism the invention includes both the cis form and the trans form as well as mixtures of these forms in all ratios. All these forms are an object of the present invention. The preparation of individual stereoisomers can be carried out, if desired, by separation of a mixture by customary methods, for example by chromatography or crystallization, by the use of stereochemically uniform starting materials for the synthesis or by stereoselective synthesis. Optionally a derivatization can be carried out before a separation of stereoisomers. The separation of a mixture of stereoisomers can be carried out at the stage of the compounds of the formula (I) or at the stage of an intermediate during the synthesis. The present invention also includes all tautomeric forms of the compounds of formula (I).

In case the compounds according to formula (I) contain one or more acidic or basic groups, the invention also comprises their corresponding pharmaceutically or toxicologically acceptable salts, in particular their pharmaceutically utilizable salts. Thus, the compounds of the formula (I), which contain acidic groups, can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I), which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) simultaneously contain acidic and basic groups in the molecule, the invention also includes, in addition to the salt forms mentioned, inner salts or betaines (zwitterions). The respective salts according to the formula (I) can be obtained by customary methods, which are known to the person skilled in the art like, for example by contacting these with an organic or inorganic acid or base in a solvent or dispersant, or by anion exchange or cation exchange with other salts. The present invention also includes all salts of the compounds of the formula (I), which, owing to low physiological compatibility, are not directly suitable for use in pharmaceuticals but which can be used, for example, as intermediates for chemical reactions or for the preparation of pharmaceutically acceptable salts.

The present invention furthermore includes all solvates of compounds of the formula (I), for example hydrates or adducts with alcohols, active metabolites of the compounds of the formula (II), and also derivatives and prodrugs of the compounds of the formula (I) which contain physiologically tolerable and cleavable groups, for example esters, amides and compounds in which the N-H group depicted in formula (I) is replaced with an N-alkyl group, such as N-methyl, or with an N-acyl group, such as N-acetyl or N-argininyl, including pharmaceutically acceptable salts formed on functional groups present in the N-acyl group.

The compounds according to general formula (I) and their precursors can be prepared according to methods published in the literature or, respectively, analogous methods. Appropriate methods have been published in, for example, Houben-Weyl, Methoden der Organischen Chemie (Methods of Organic Chemistry), Thieme-Verlag, Stuttgart, or Organic Reactions, John Wiley & Sons, New York.

All reactions for the synthesis of the compounds of the formula (I) are per se well-known to the skilled person and can be carried out under standard conditions according to or analogously to procedures described in the literature, for example in Houben-Weyl, Methoden der Organischen Chemie (Methods of Organic Chemistry), Thieme-Verlag, Stuttgart, or Organic Reactions, John Wiley & Sons, New York. Depending on the circumstances of the individual case, in order to avoid side reactions during the synthesis of a compound of the formula (I), it can be necessary or advantageous to temporarily block functional groups by introducing protective groups and to deprotect them in a later stage of the synthesis, or introduce functional groups in the form of precursor groups which in a later reaction step are converted into the desired functional groups. Such synthesis strategies and protective groups and precursor groups, which are suitable in an individual case, are known to the skilled person. If desired, the compounds of the formula (I) can be purified by customary purification procedures, for example by recrystallization or chromatography. The starting compounds for the preparation of the compounds of the formula (I) are commercially available or can be prepared according to or analogously to literature procedures. The compounds obtained with the above-mentioned synthesis methods are a further object of the present invention.

The compounds according to the formula (I) can also be used in combination with other pharmaceutically active compounds, preferably compounds which are able to enhance the effect of the compounds according to the general formula (I). Examples of such compounds include: (i) antimetabolites, cytarabine, fludarabine, 5-fluoro-2'-deoxyuridine, gemcitabine, hydroxyurea or methotrexate; (ii) DNA-fragmenting agents, bleomycin, (iii) DNA-crosslinking and alkylating agents, chlorambucil, cisplatin, carboplatin, fotemustine, cyclophosphamide, ifosfamide, dacarbazine or nitrogen mustard; (iv) intercalating agents, adriamycin (doxorubicin) or mitoxantrone; (v) protein synthesis inhibitors, L-asparaginase, cycloheximide, puromycin or diphteria toxin; (vi) topoisomerase I poisons, camptothecin or topotecan; (vii) topoisomerase II poisons, etoposide (VP-16) or teniposide; (viii) microtubule-directed agents, colcemid, colchicine, paclitaxel (taxol), docetaxel (taxotere), vinblastine or vincristine; (ix) kinase inhibitors, flavopiridol, staurosporin, STI571 (CPG 57148B) or UCN-01 (7-hydroxystaurosporine); (x) miscellaneous investigational agents, trichostatin A, thioplatin, PS-341, phenylbutyrate, ET-18-OCH₃, or farnesyl transferase inhibitors (L-739749, L-744832); polyphenols, quercetin, resveratrol, piceatannol, epigallocatechine gallate, theaflavins, flavanols, procyanidins, betulinic acid and derivatives thereof; (xi) hormones, glucocorticoids or fenretinide; (xii) hormone antagonists, tamoxifen, finasteride or LHRH antagonists, (xiii) demethylating agents, 5-azacytidine, 5-aza-2'deoxycytidine, 5,6-dihydro-5-azacytidine, or (xiv) a combination of any of the pharmaceuticals given above or use in high-dose chemotherapy regimens including stem cell transplantation; (xv) differentiation inducing agents such as retinoic acid derivatives; (xvi) ionizing radiation therapy, MIBG-therapy and conventional radiation therapy.

The compounds of the formula (I) and their pharmaceutically acceptable salts, optionally in combination with other pharmaceutically active compounds, can be administered to animals, preferably to mammals, and in particular to humans, as pharmaceuticals by themselves, in mixtures with one another or in the form of pharmaceutical preparations. Further subjects of the present invention therefore also are the compounds of the formula (I) and their pharmaceutically acceptable salts for use as pharmaceuticals, their use as inhibitors of DNMTs and/or DNA methylation, and in particular their use in the therapy and prophylaxis of the above-mentioned syndromes as well as their use for preparing pharmaceuticals for these purposes. Furthermore, subjects of the present invention are pharmaceutical preparations (or pharmaceutical compositions), which comprise an effective dose of at least one compound of the formula (I) and/or a pharmaceutically acceptable salt thereof and a pharmaceutically acceptable carrier, i.e. one or more pharmaceutically acceptable carrier substances and/or additives.

The pharmaceuticals according to the invention can be administered orally, for example in the form of pills, tablets, lacquered tablets, sugar-coated tablets, granules, hard and soft gelatin capsules, aqueous, alcoholic or oily solutions, syrups, emulsions or suspensions, or rectally, for example in the form of suppositories. Administration can also be carried out parenterally, for example subcutaneously, intramuscularly or intravenously in the form of solutions for injection or infusion. Other suitable administration forms are, for example, percutaneous or topical administration, for example in the form of ointments, tinctures, sprays or transdermal therapeutic systems, or the inhalative administration in the form of nasal sprays or aerosol mixtures, or, for example, microcapsules, implants or rods. The preferred administration form depends, for example, on the disease to be treated and on its severity.

The preparation of the pharmaceutical preparations can be carried out in a manner known per se. To this end, one or more compounds of the formula (I) and/or their pharmaceutically acceptable salts, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form, which can then be used as a pharmaceutical in human or veterinary medicine.

For the production of pills, tablets, sugar-coated tablets and hard gelatin capsules it is possible to use, for example, lactose, starch, for example maize starch, or starch derivatives, talc, stearic acid or its salts, etc. Carriers for soft gelatin capsules and suppositories are, for example, fats, waxes, semisolid and liquid polyols, natural or hardened oils, etc. Suitable carriers for the preparation of solutions, for example of solutions for injection, or of emulsions or syrups are, for example, water, physiologically sodium chloride solution, alcohols such as ethanol, glycerol, polyols, sucrose, invert sugar, glucose, mannitol, vegetable oils, etc. It is also possible to lyophilize the compounds of the formula (I) and their pharmaceutically acceptable salts and to use the resulting lyophilisates, for example, for preparing preparations for injection or infusion. Suitable carriers for microcapsules, implants or rods are, for example, copolymers of glycolic acid and lactic acid.

Besides the compound or compounds according to the invention and carriers, the pharmaceutical preparations can also contain additives, for example fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The dosage of the compound of the formula (I) to be administered and/or of a pharmaceutically acceptable salt thereof depends on the individual case and is, as is customary, to be adapted to the individual circumstances to achieve an optimum effect. Thus, it depends on the nature and the severity of the disorder to be treated, and also on the sex, age, weight and individual responsiveness of the human or animal to be treated, on the efficacy and duration of action of the compounds used, on whether the therapy is acute or chronic or prophylactic, or on whether other active compounds are administered in addition to compounds of the formula (I). The daily dose can be administered in a single dose or, in particular when larger amounts are administered, be divided into several, for example two, three or four individual doses. In some cases, depending on the individual response, it may be necessary to deviate upwards or downwards from the given daily dose.

Finally, the present invention relates to a method of identifying a compound, which induces apoptosis. The method comprises the steps of (a) contacting a compound suspected to induce apoptosis with a dividing cell and (b) determining whether the compound affects the distribution of the chromosomes in the daughter cells, i.e. numeric aberrations in the daughter cells are to be determined and/or (c) preferably determining whether the compound affects the structure, chemical modification and spacial distribution of chromatin. A numeric aberration, which occurs in a daughter cell is indicative for compound, which is capable of inducing apoptosis. The compound, preferably, elicits its therapeutic effects on dividing cells, such as tumour cells, due to its capability of inducing numeric aberrations which will result in subsequent apoptosis of the daughter cells carrying said numeric aberrant chromosomes.

Suitable cells to be used for the aforementioned methods are preferably fast dividing cells, more preferably tumour cells and, most preferably, the tumour cells referred to in this specification and the accompanying Examples.

Numeric aberrations can be easily detected by analysing the karyogram of the daughter cells. Numeric aberrations may result in trisomy (i.e. three times a chromosome instead of twice) or monosomy (i.e. a chromosome is present only once instead of twice). Other numeric aberrations may occur, of course, as well.

Suitable compounds to be screened encompass organic or inorganic molecules, small molecules, metabolites or precursors thereof, nucleic acids (DNA and RNA), nucleotides and derivative thereof, peptides, proteins, amino acids and derivatives thereof. More preferably, the compounds to be used in the method of the present invention are selected from those specified above or those disclosed in WO 2005/039498.

It is to be understood that the compounds identified in accordance with the method of the present invention may be used as pharmaceutical compositions to treat neuroblastomas and, preferably, even the advanced stage neuroblastomas as referred to elsewhere in this specification. Accordingly, the method of the present invention may further comprise the steps of manufacturing the identified compound as a pharmaceutical composition as described elsewhere in this specification.

The figures show:

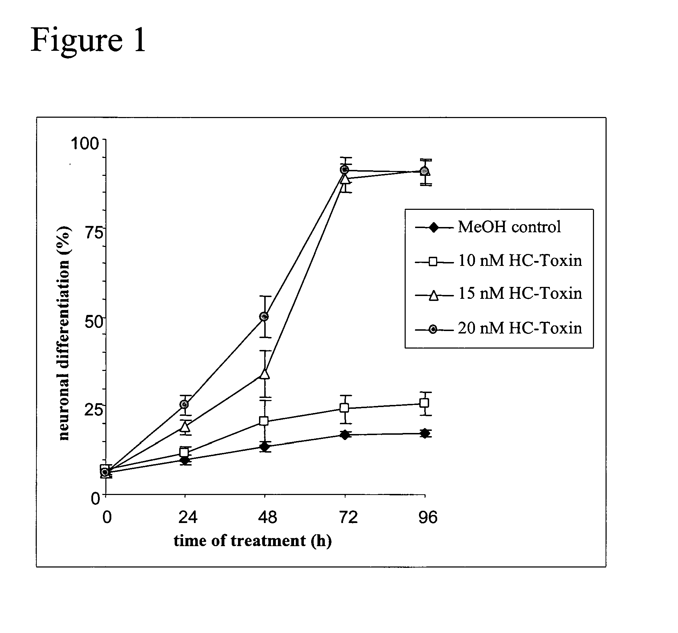

**Figure 1:** HC-Toxin induces neuronal differentiation in SH-EP *MYCN*-single copy neuroblastoma cells. Cells with neurites longer than twice the diameter of the cell body have been deemed as differentiated cells. The figure shows the mean including standard deviations of three independent experiments carried out in triplicates.

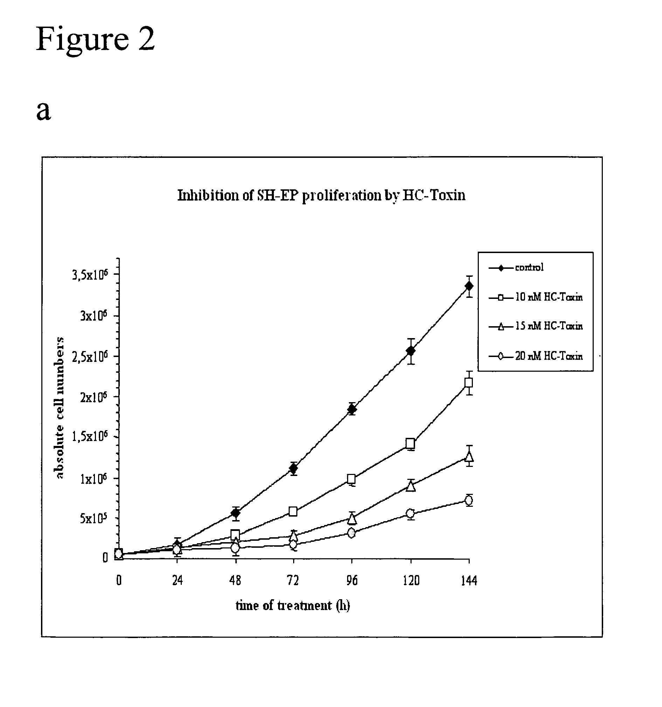

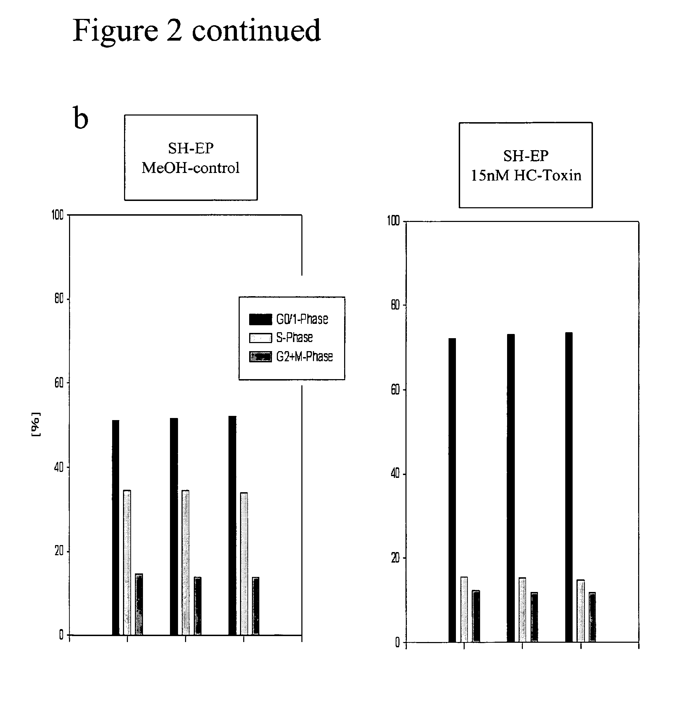

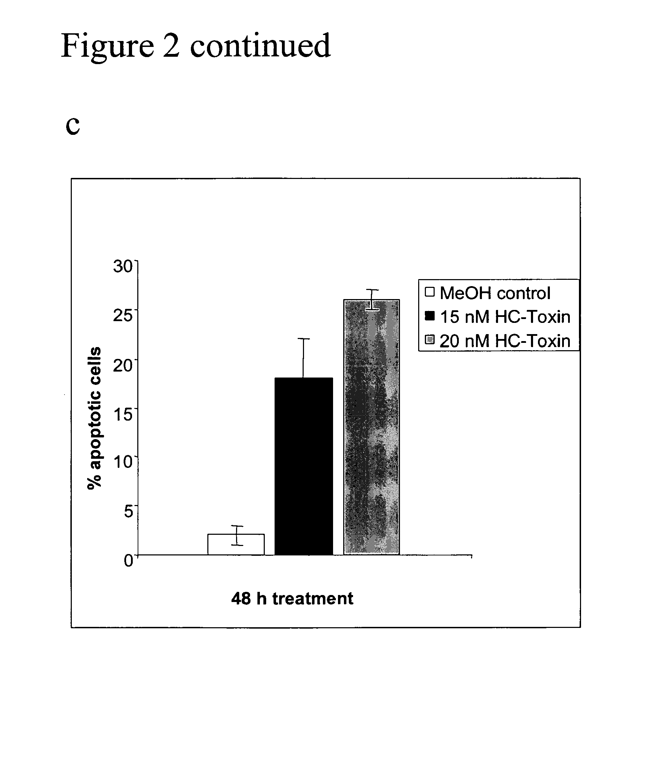

**Figure 2:** Influence of HC-Toxin upon proliferation, cell cycle, and apoptosis of SH-EP neuroblastoma cells. (a) HC-Toxin (10 to 20 nM) inhibits cell proliferation in a concentration dependent manner. (b) HC-Toxin results in an accumulation of cells in G0/1 phase of the cell cycle. (c) HC-Toxin induces apoptosis in a concentration dependent manner. The figure shows the mean including standard deviations of three experiments.

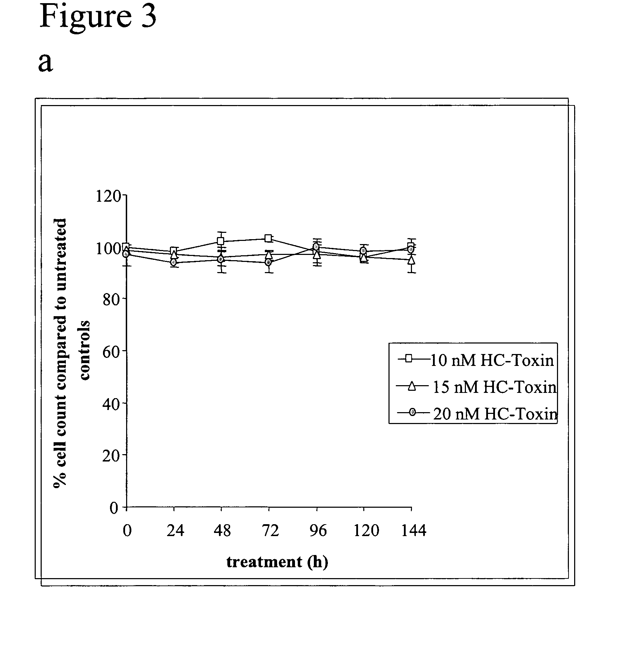

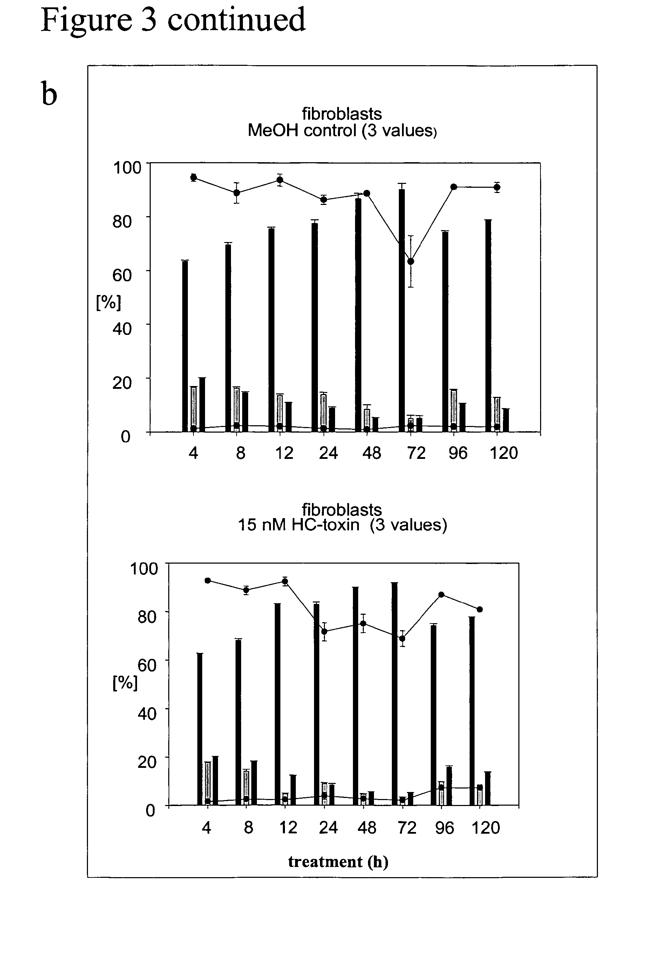

**Figure 3:** Influence of HC-Toxin upon proliferation and cell cycle of primary non-transformed skin fibroblasts. (a) HC-toxin (10-20 nM) has no influence upon the proliferation of primary non-transformed skin fibroblasts. (b) HC-Toxin does not alter the cell cycle of primary non-transformed skin fibroblasts Exemplarily presented are the results obtained when treating fibroblasts with 15 nM HC-toxin compared to solvent treated (Methanol, MeOH) controls. The figure shows the mean including standard deviations of three independent experiments.

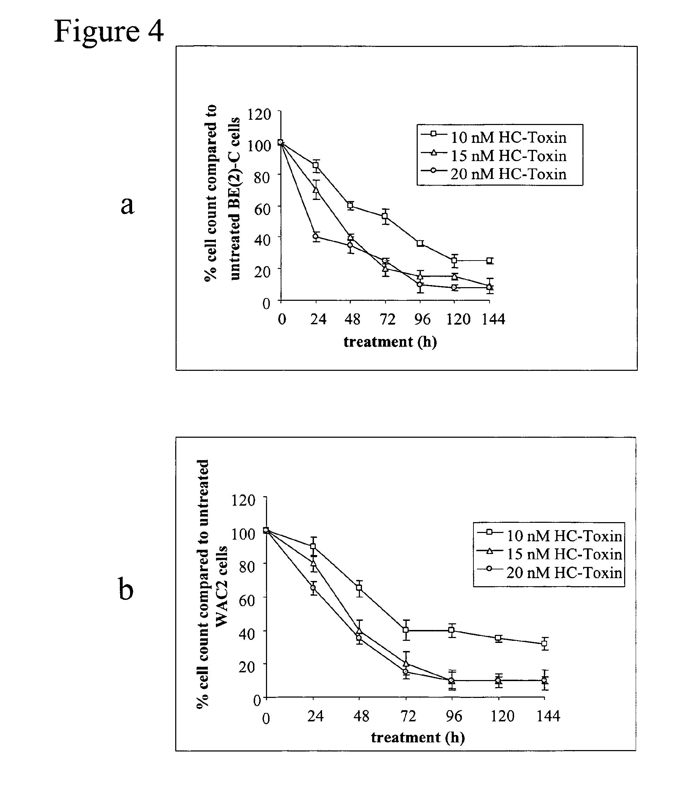

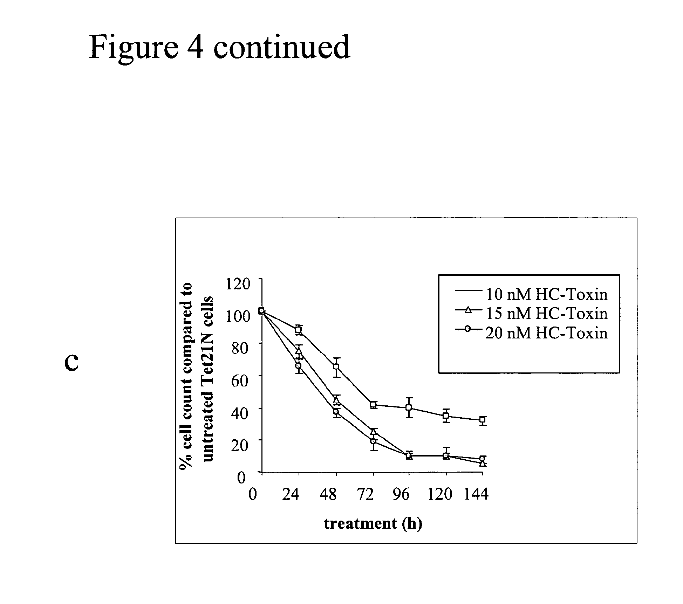

**Figure 4:** Influence of HC-Toxin upon the proliferation of *MYCN* amplified neuroblastoma cells and cells, which overexpress *MYCN.* (a) BE(2)-C neuroblastoma cells, (b) WAC2 neuroblastoma cells, and (c) Tet21N neuroblastoma cells.

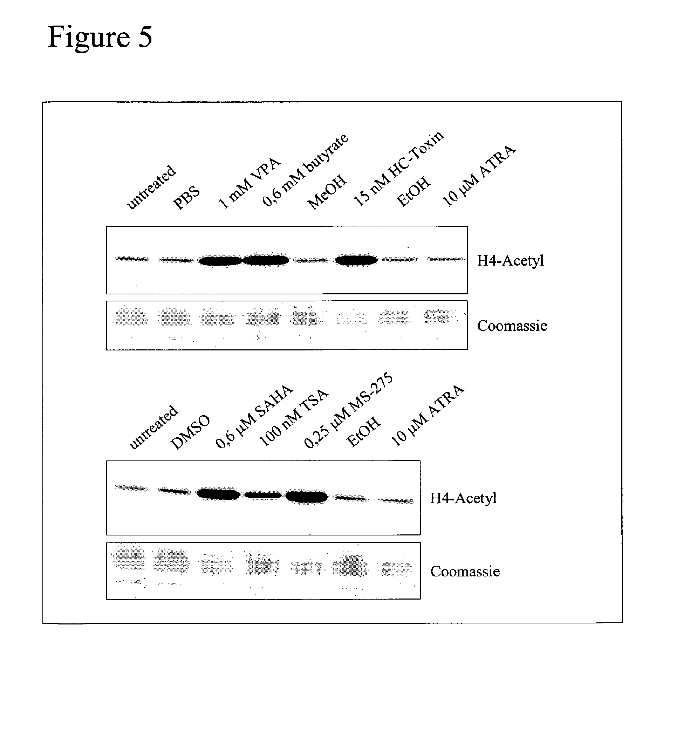

**Figure 5**: HC-Toxin induces hyperacetylation of histone H4 via inhibition of nuclear histone deacetylases at nanomolar concentrations in SH-EP neuroblastoma cells. The cells were treated for 6 hours with HC-toxin or the indicated histone deacetylase inhibitors as well as *all-trans* retinoic acid. Whole-cell extracts were obtained and subjected to Western-Blot analysis using an antibody, which specifically recognizes acetylated histone H4 (H4 Acetyl).

The invention will now be illustrated by the following Examples. However, the Examples are not meant to limit the scope of the invention in any respect.

### Example 1: HC-Toxin induces neuronal differentiation of neuroblastoma cells.

5x10*4 SH-EP neuroblastoma cells were cultured in RPMI 1640 medium (including 10 % FBS, 1 % Pen/Strep). After 24 hours of culture, the cells were treated up to 96 h with 10 to 20 nM HC-Toxin or, as solvent control, with methanol (MeOH) at 37°C, 5 % CO2. The morphological changes of the cells were investigated using phase contrast microscopy. After 36 h of treatment, neurite outgrowth was observed. After 72 h of treatment, said neurite outgrowth reached its maximum and was constant if treatment was prolonged (not shown). Immunocytochemical analysis on protein level showed expression of dendritic (MAP2) as well as axonal (neurofilament 3, 150 kD) differentiation markers (not shown). More than 95 % of the SH-EP neuroblastoma cells differentiate morphologically when treated with 15 nM HC-Toxin (see Figure 1c).

### Example 2: HC-Toxin induces inhibition of proliferation, growth arrest, and apoptosis in SH-EP neuroblastoma cells.

Figure 2a shows the concentration-dependent inhibition of SH-EP neuroblastoma cell proliferation due to HC-Toxin. A comparison of HC-Toxin treated cells (15 nM) with control cells revealed accumulation of the HC-Toxin treated cells in the G0/1 phase of the cell cycle (Figure 2b). An increase in apoptotic cells from 5 % to 20 % was observed after 48 h to 72 h of HC-toxin treatment in a dose dependent manner (Figure 2c).

### Example 3: HC-Toxin has no effect upon "normal" non-transformed cells of the human body.

In order to determine the effects of HC-Toxin on "normal" body cells, primary skin fibroblasts were treated under the same conditions. No significant changes on proliferation or cell cycle were observed (see Figure 3).

### Example 4: HC-Toxin antagonizes the effect of MYCN oncogene upon proliferation of neuroblastoma cells.

*MYCN* amplified neuroblastoma cells (Be(2)-C), MYCN stably transfected SH-EP-neuroblastoma cells (WAC2) as well as SH-EP cells having an tetracycline inducible *MYCN* (Tet21N) were treated by HC-Toxin. Figure 4 shows that HC-Toxin efficiently inhibits proliferation of *MYCN* amplified cells as well as neuroblastoma cells overexpressing MYCN. Comparison revealed a stronger inhibition of proliferation in *MYCN* amplified cells (BE(2)-C, WAC2, Tet21N) than in *MYCN* single copy cells (SH-EP).

### Example 5: HC-Toxin inhibits nuclear histone deacetylases in neuroblastoma cells in nanomolar concentrations.

SH-EP-neuroblastoma cells were treated with 15 nM HC-Toxin for 6 h. Subsequently, accumulation of acetylated histone H4 was investigated. In comparison to other histone deacetylase inhibitors, iHC-Toxin was found to induce accumulation of acetylated histone H4 even in 10*3 to 10*6-fold lower concentrations (Figure 5). In contrast, *all-trans* retinoic acid, which is being used as differentiation inducing agent for the therapy of high-risk neuroblastoma, induced no induction of hyperacetylation of histone H4 in SH-EP neuroblastoma cells.

Cells and reagents referred to above are available or were purchased from: HC-Toxin (Sigma, # H7270), Methanol (Roth), SH-EP neuroblastoma cells (DKFZ), BE(2)-C neuroblastoma cells (ECACC, No. 95011817), WAC2 neuroblastoma cells (Dr. Frank Westermann, DKFZ), Tet21N neuroblastoma cells (Dr. Frank Westermann, DKFZ), primary skin fibroblasts (DKFZ), RPMI 1640 (Cambrex), DMEM with L-Glutamin (Cambrex), penicillin/ Streptomycin (Cambrex), Trypsin/EDTA (Cambrex), fetal bovine serum (Biochrom), DPBS (Cambrex), "tissue culture treated plastics" (Greiner, BD Falcon), anti-acetyl-Histone H4 antibody (Upsate), donkey-anti-rabbit IgG (Promega), full range rainbow protein marker (Amersham), anti-Neurofilament 159 (Chemicon), anti-MAP2 (Chemicon), anti-dopamine (Chemicon), anti-tyrosine-hydroxylase (Chemicon), anti-synaptophysin (Chemicon), anti-synapsin 1 (Chemicon), paraformaldehyde (Sigma), Triton-X-100 (Sigma).

## Claims

1. A compound according to the general formula (I) 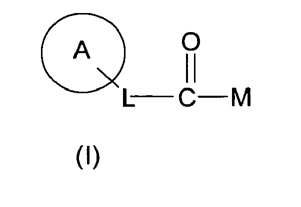 wherein the symbols have the following meanings:
A is a 9- to 15-membered heterocycle optionally carrying one or more double bonds, and comprising 3 to 5 units of the type -D-E-G- wherein
D is selected from -N(R¹)-, -O-, and -S(O)(R²R³)ₘ-; and/or
E is selected from methylene -CH₂-, ethylene -CH₂CH₂- and propylene -CH₂-CH₂-CH₂-, wherein in the methylene, ethylene and propylene groups one or more H atoms can be replaced by: linear and branched C₁- to C₁₀ alkyl groups, which are optionally substituted by one or more substituents from the group -OH, F, -SH, -SCH₃, -NH₂, - NHC(NH)NH₂, -C(O)OH, -C(O)NH₂, C₆H₅, C₆H₄OH, indolyl, imidazolyl or a salt thereof; and linear and branched C₂- to C₁₀ alkylene groups, which are optionally substituted by one or more OH and/or one or more F; or, in case 2 or more even-numbered substituents are present, the named alkyl or alkylene group forms together with a further alkyl or alkylene group on the same or a different carbon atom a 5- to 7-membered cycle; or the named alkyl and alkylene groups form together with the group R¹ a 5- to 7-membered cycle which includes the C and the N atom to which the named groups are attached; and/or
G is a keto group -C(O)- or a thioketo group -C(S)-; and/or
wherein the units -D-E-G- not attached to L can be fully or partially identical with or entirely different from each other; and/or
the cycle A can comprise one or more double bonds; and/or
L is attached to D or E in one unit -D-E-G- and is a linear C₃- to C₅-alkyl group in which one or more carbon atoms may be replaced by non adjacent O atoms, and wherein one or more H atom in the alkyl chain may be substituted by one or more alkyl groups; and/or
M is selected from epoxy groups 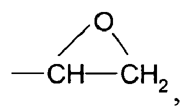 hydroxyamino groups -N(OH)H, hydroxymethyl groups -CH₂OH, 1-fluoroethyl groups -CHFCH₃ and 1-chloroethyl groups -CHClCH₃; and/or
R¹ is selected from: H; linear and branched C₁- to C₁₀ alkyl groups, which are optionally substituted by one or more OH and/or one or more F; and linear or branched C₂- to C₁₀ alkylene groups which are optionally substituted by one or more OH and/or one or more F; or R¹ forms together with one alkyl or alkylene group in E a 5- to 7-membered cycle which includes the C and the N atom to which the named groups are attached; and/or
R² and R³ are independently of each other selected from: H; linear and branched C₁- to C₁₀ alkyl groups which are optionally substituted by one or more OH and/or one or more F; and linear and branched C₂- to C₁₀ alkylene groups, which are optionally substituted by one or more OH and/or one or more F; or R² and R³, together with the sulfur atom to which they are attached, form a 5- to 7-membered cycle; or one of R², R³ forms together with one alkyl or alkylene group in E a 5- to 7-membered cycle, which includes the C and the S atom to which the named groups are attached; and/or
m is 0 or 1,
and/ or a pharmaceutically acceptable salt thereof,
with the proviso that HC toxin is excluded.

2. A compound according to claim 1, wherein the cycle A comprises 3, 4 or 5 amino acids in the D- or L-form

3. A compound according to claim 1 or 2, wherein the substituents have the following meanings:
A is a 11- to 13-membered cycle and comprises 4 units of the type -D-E-G- , wherein
D is NR¹; and/or
E is -CH₂-, wherein one or both H atoms can be replaced by: a linear or branched C₁-to C₄ alkyl group, which are optionally substituted by one or more substituents from the group -OH, F, -SH, -SCH₃, -NH₂, -NHC(NH)NH₂, -C(O)OH, -C(O)NH₂, C₆H₅, C₆H₄OH, indolyl, imidazolyl or a salt thereof; and/or a linear or branched C₂- to C₄ alkylene group, which is optionally substituted by one or more OH and/or one or more F; or the named alkyl and alkylene groups form together with the group R¹ a 5- to 7-membered cycle which includes the C and the N atom to which the named groups are attached; and/or
G is a keto group -C(O)-; and/or
wherein the units -D-E-G- not attached to L can be fully or partially identical with or entirely different from each other; and/or
the cycle A can comprise one or more double bonds; and/or
R¹ is selected from: H; linear and branched C₁- to C₄ alkyl groups, which are optionally substituted by one or more OH and/or one or more F; and linear and branched C₂- to C₄ alkylene groups which are optionally substituted by one or more OH and/or one or more F; or R¹ forms together with one alkyl or alkylene group in E a 5- to 7-membered cycle, which includes the C and the N atom to which the named groups are attached, and/or
L is attached to E in one unit -D-E-G- and is a linear C₃- to C₅-alkyl group in which one or more carbon atoms may be replaced by non adjacent O atoms, and wherein one or more H atom in the alkyl chain may be substituted by one or more alkyl groups; and/or
M is selected from epoxy groups 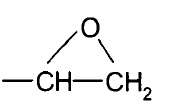
and hydroxyamino groups -N(OH)H.

4. A compound according to claim 3, wherein the cycle A comprises 4 natural amino acids in the L- or D-form.

5. A compound according to claim 3 or 4, wherein the substituents have the following meanings:
A is a 12-membered cycle and comprises 4 units of the type -D-E-G- , wherein
D is NR¹; and/or
E is -CH₂-, wherein in one or two units -D-E-G-, one or both H atoms can be replaced by: a linear or branched C₁- to C₄ alkyl group and/or a linear or branched C₂- to C₄ alkylene group; and wherein in only one unit -D-E-G-, one H atom in -CH₂- is replaced by a linear or branched C₁- to C₄ alkyl group or a linear or branched C₂- to C₄ alkylene group, with the said alkyl or alkylene group forming together with R¹ in the neighbouring NR¹ group a 5- to 7-membered cycle, which includes the C and the N atom to which the named groups are attached; and/or
G is a keto group -C(O)-; and/or
at least 2 units -D-E-G- are different from each other; and/or
the cycle A can comprise one or more double bonds; and/or
R¹ is selected from: H; linear and branched C₁- to C₄ alkyl groups, which are optionally substituted by one or more OH and/or one or more F; and linear and branched C₂- to C₄ alkylene groups, which are optionally substituted by one or more OH and/or one or more F; and wherein in exactly one unit -D-E-G-, R¹ forms together with the said alkyl or alkylene in the neighbouring -CH₂- group a 5- to 7-membered cycle, which includes the C and the N atom to which the named groups are attached; and/or
L is attached to E in one unit -D-E-G- and is a linear C₃- to C₅-alkyl group, and wherein one or more H atom in the alkyl chain may be substituted by one or more alkyl groups; and/or
M is selected from epoxy groups 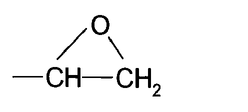 and hydroxyamino groups -N(OH)H.

6. A compound according to claim 5, wherein the substituents have the following meanings:
A is a 12-membered cycle and comprises 4 units of the type -D-E-G- , wherein
D is NR¹; and/or
E is -CH₂-, wherein in one or two units -D-E-G-, one or both H atoms can be replaced by: a methyl or ethyl group; and wherein in only one unit -D-E-G-, one H atom in - CH₂- is replaced by a linear or branched C₁- to C₄ alkyl group or a linear or branched C₂- to C₄ alkylene group, and wherein the said alkyl or alkylene group forms together with R¹ in the neighbouring NR¹ group a 5- to 7-membered cycle which includes the C and the N atom to which the named groups are attached;
G is a keto group -C(O)-; and/or
wherein at least 2 units -D-E-G- are different from each other; and/or
the cycle A can comprise one or more double bonds; and/or
R¹ is H or a linear or branched C₁- to C₄-alkyl group in three of the units -D-E-G-; and wherein in the other unit -D-E-G, R¹ forms together with the said alkyl or alkylene in the neighbouring -CH₂- group a 5- to 7-membered cycle, which includes the C and the N atom to which the named groups are attached; and/or
L is attached to E in one unit -D-E-G- and is a C₃- to C₅-alkyl group, and wherein one or more H atom in the alkyl chain may be substituted by one or more alkyl groups; and/or
M is selected from epoxy groups 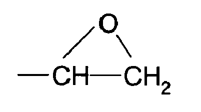 and hydroxyamino groups -N(OH)H.

7. A compound as defined in any of the claims 1 to 6, or a pharmaceutically acceptable salt thereof, for use as a medicament.

8. Use of a compound as defined in any of the claims 1 to 6, or a pharmaceutically acceptable salt thereof, for the manufacture of a pharmaceutical composition for treating a neuroblastoma.

9. The use of claim 8, wherein said neuroblastoma is an advanced stage neuroblastoma.

10. The use of claim 9, wherein said advanced stage neuroblastoma is a stage IV neuroblastoma.

11. The use of any one of claims 9 to 10, wherein the MYCN oncogene is amplified in the cells said neuroblastoma.

12. Method for treating a neuroblastoma in a patient comprising administering to a patient suffering from said disease in a therapeutically effective amount a compound as defined in any of claims 1 to 6.

13. A pharmaceutical composition for the treatment of neuroblastoma, comprising a therapeutically effective amount a compound as defined in any of claims 1 to 6, and optionally carriers and additives.
